Europäisches Patentamt

European Patent Office

Office européen des brevets

(11) Numéro de publication : **0 334 710 B1**

(19)

(12) **FASCICULE DE BREVET EUROPEEN**

(45) Date de publication du fascicule du brevet :
**22.04.92 Bulletin 92/17**

(21) Numéro de dépôt : **89400666.7**

(22) Date de dépôt : **09.03.89**

(51) Int. Cl.⁵ : **B01J 19/24,** B01J 12/00,
B01F 5/00, C07C 29/15,
// C01B3/34, C07C253/24

(54) **Procédé d'oxydation d'une charge oxydable en phase gazeuse et réacteur pour la mise en oeuvre de ce procédé.**

(30) Priorité : **16.03.88 FR 8803495**

(43) Date de publication de la demande :
**27.09.89 Bulletin 89/39**

(45) Mention de la délivrance du brevet :
**22.04.92 Bulletin 92/17**

(84) Etats contractants désignés :
**DE GB IT NL**

(56) Documents cités :
**EP-A- 0 231 706**
**FR-A- 2 304 601**

(73) Titulaire : **INSTITUT FRANCAIS DU PETROLE**
**4, Avenue de Bois Préau**
**F-92506 Rueil-Malmaison Cédex (FR)**

(72) Inventeur : **Alagy, Jacques**
**24, Chemin Bekensteiner**
**F-69260 Charbonnieres (FR)**
Inventeur : **Busson, Christian**
**11, Chemin du Cogny**
**F-69570 Dardilly (FR)**
Inventeur : **Broutin, Paul**
**28, allée Simon Saint Jean**
**F-69139 Ecully (FR)**
Inventeur : **Weill, Jérôme**
**16, route des Tourelles**
**F-69005 Lyon (FR)**

EP 0 334 710 B1

Jouve, 18, rue Saint-Denis, 75001 PARIS

## Description

La présente invention concerne un procédé d'oxydation d'une charge oxydable en phase gazeuse par un gaz ou un mélange de gaz comprenant au moins un gaz oxydant c'est à dire un gaz permettant l'oxydation de ladite charge.

La présente invention concerne également l'appareillage nécessaire à la mise en oeuvre dudit procédé.

La présente invention concerne plus particulièrement un procédé d'oxydation, de préférence lente et habituellement partielle, de charges oxydables telles que par exemple de charges hydrocarbonées, en vue notamment de la préparation de gaz de synthèse comprenant du monooxyde de carbone et de l'hydrogène pour la synthèse, par exemple de méthanol et d'alcools homologues supérieurs.

Ce procédé peut s'appliquer, par exemple, à l'oxydation des effluents de réformage à la vapeur, du benzène, ou aux réactions d'ammoxydation.

Bien que les gaz oxydants puissent être notamment l'oxygène, l'ozone ou les halogènes, on ne considérera à titre d'exemple, dans la présente invention, que les réactions avec l'oxygène.

Selon le brevet FR 2304601 relatif à un procédé d'ammonoxydation, l'oxygène est distribué dans une couche fixe catalytique en une multiplicité de niveaux pour réduire l'exothermicité.

Il est connu selon le brevet US-A-2621117 de réaliser une oxydation partielle du méthane. La réaction se fait dans une flamme où le mélange des gaz n'est jamais parfait et on atteint rapidement des températures très élevées dans les zones riches en oxygène.

Les gaz, produits à haute température sont ensuite mélangés avec la majeure partie de la charge à oxyder et provoquent le craquage des molécules avec formation de carbone susceptible, par exemple, d'encrasser des catalyseurs dans la suite du procédé et donc de diminuer le rendement de la réaction.

Dans le cas du méthane, on observe une production de carbone et les gaz de synthèse doivent être ultérieurement dépoussiérés avant leur utilisation, par exemple pour la synthèse d'alcools à partir du monoxyde de carbone et de l'hydrogène.

Selon ce document, l'oxygène est injecté à travers un seul canal qui doit avoir une section suffisante pour admettre la totalité du débit et bien que le gaz soit injecté à grande vitesse à travers cette section, la vitesse de dispersion des molécules est lente, comparée à la vitesse de réaction en phase gazeuse, et il peut se produire, en plus de la formation de noir de carbone, une surchauffe excessive au niveau de la zone où s'effectue le contact des gaz réactionnels, ce qui est préjudiciable au bon fonctionnement du procédé.

De plus, le jet d'oxygène à l'endroit où il sort de son orifice est habituellement dans l'environnement du gaz à oxyder qui circule à faible vitesse dans le réacteur, ce qui n'est pas favorable à une dispersion rapide des molécules d'oxygène.

La demande de brevet européen EP-A-231706, au nom de la demanderesse, décrit un procédé d'oxydation d'une charge oxydable en phase gazeuse par un mélange de gaz contenant au moins un gaz oxydant dans lequel les gaz réactionnels sont distribués par une multitude de conduits parallèles séparés jusqu'à l'entrée d'une zone de mélange assurant un mélange rapide desdits gaz, ledit mélange étant ensuite envoyé dans une zone de réaction comprenant des dispositifs d'arrêt ou coincement de flamme permettant à la réaction de s'effectuer sans explosion, bien que l'on soit le plus souvent à l'intérieur des limites explosives (notamment dans le cas de l'oxydation partielle du méthane).

Dans ce procédé la vitesse des gaz mesurée à la sortie du distributeur ou à la sortie de la zone de mélange est habituellement relativement faible, compte tenu du débit des gaz pour un réacteur donné, corrigé des effets de pression et de température, ce qui contribue à un mélange des gaz hétérogène à la sortie de la zone de mélange d'autant plus que le mélangeur statique utilisé dans ces conditions n'est en général pas d'une très grande efficacité. Il s'ensuit la formation de suies qui sont préjudiciables à la bonne marche du procédé.

Par ailleurs le procédé décrit dans ce brevet ne permet pas d'obtenir de bons résultats lorsque l'on désire effectuer les réactions d'oxydation à pression élevée, ce qui est particulièrement souhaitable, en particulier dans le cas où le mélange de gaz oxydé est ensuite utilisé par exemple pour la synthèse d'alcools.

Un des objets de l'invention est de remédier aux inconvénients ci-dessus.

On a découvert et ceci constitue l'objet de la présente invention un procédé d'oxydation d'une charge oxydable en phase gazeuse par un gaz ou un mélange de gaz oxydant permettant d'obtenir des résultats sensiblement améliorés vis à vis des procédés de l'art antérieur et en particulier vis à vis du procédé décrit par la demanderesse dans la demande de brevet EP-A-231706.

De façon plus précise, la présente invention concerne un procédé d'oxydation d'une charge oxydable en phase gazeuse par un gaz ou un mélange de gaz, comprenant au moins un gaz oxydant, comprenant les étapes suivantes :

a) on introduit la charge oxydable et le gaz oxydant dans une zone de dispersion entièrement remplie d'éléments particulaires en matière céramique ayant des dimensions telles que les espaces ou passages libres

2

EP 0 334 710 B1

entre lesdits éléments ou passages entre les parois de ces éléments, aient, suivant au moins une direction, une dimension au plus égale à la distance de coincement de la flamme pouvant résulter de l'oxydation de ladite charge par ledit gaz oxydant, lesdits gaz étant introduits dans ladite zone de dispersion à l'aide d'au moins un moyen d'accélération des gaz, noyé dans lesdits éléments particulaires, permettant d'obtenir une vitesse des gaz à la sortie dudit moyen d'accélération d'au moins 10 m x s $^{-1}$, et

b) on fait réagir le mélange gazeux issu de l'étape (a) dans une zone de réaction en matière céramique comprenant une multiplicité d'espaces présentant des passages ayant, suivant au moins une direction, une dimension au plus égale à ladite distance de coincement de flamme, la distance inter-zones étant au plus égale à ladite distance de la coincement de flamme.

Le procédé de la présente invention présente les avantages suivants :

– il permet d'obtenir à l'entrée de la zone de réaction un mélange très homogène de charge oxydable et de gaz oxydant en raison de l'énergie communiquée aux gaz qui contribue à une efficacité accrue de la zone de mélange ;

– la répartition du mélange gazeux sur l'ensemble de la surface de la section du réacteur est particulièrement bonne, ce qui permet en particulier à la réaction de se dérouler dans tout le volume de la zone de réaction et évite ainsi largement l'apparition de zones localement surchauffées ;

– l'utilisation de moyens permettant l'arrêt ou coincement de la flamme pouvant résulter de l'oxydation de la charge par le gaz oxydant, évite l'explosion et permet cependant d'opérer à des températures pouvant dépasser 1000 °C, tout en protégeant le réacteur et les divers dispositifs de la chaleur souvent excessive dégagée lors de l'oxydation, habituellement partielle, de la charge ;

– par ailleurs l'utilisation de moyens d'accélération dans lesquels on introduit la charge oxydable et le gaz oxydant, permet en particulier un meilleur contrôle des températures dans les diverses zones du réacteur, principalement à proximité de l'entrée des gaz dans la zone de réaction, par simple variation de la vitesse des gaz à la sortie desdits moyens d'accélération.

Le procédé de la présente invention permet de travailler sous pression élevée sans formation excessive de suies. La pression utilisée est habituellement d'environ 1,5 MPa à 25 MPa, de préférence d'environ 2MPa à 20 MPa et le plus souvent d'environ 2 MPa à 10 MPa. Selon le procédé de la présente invention il est également possible d'utiliser de l'air ou de l'air enrichi en oxygène, sans inconvénients notables, en particulier sans formation excessive de suies, ce que ne permet pas le procédé décrit dans la demande de brevet EP-A-231706 dans laquelle la formation de suies n'est évitée que si l'on utilise de l'oxygène pur et si l'on travaille à faible pression, par exemple 1 MPa.

Le gaz oxydant et la charge oxydable peuvent être introduits ensemble dans la zone d'accélération de la zone de dispersion ou avantageusement de manière séparée. Dans ce dernier cas, le gaz peut être introduit dans un moyen d'accélération et la charge dans un autre, donc en alternance lorsque le nombre des accélérateurs est élevé. Ces derniers seront de préférence situés sur un même plan perpendiculaire à la direction de l'écoulement de la zone de dispersion, et selon une répartition symétrique par rapport à l'axe longitudinal du réacteur.

Le nombre d'accélérateurs de vitesse de ces gaz dépend de la taille du réacteur et de la quantité de fluide à introduire par unité de temps. Il sera avantageusement attribué un nombre d'accélérateurs plus important à la charge oxydable qu'au gaz oxydant, par exemple selon un rapport 4 : 1.

Les conditions d'opération et les moyens d'accélération sont généralement choisis de manière à ce que la vitesse des gaz à la sortie desdits moyens soit d'au moins 10 m x s$^{-1}$ à environ 330 m x s$^{-1}$ ou même supérieure, avantageusement de 20 à 200 m x s$^{-1}$ et de préférence de 50 à 150 m x s$^{-1}$.

Les éléments particulaires remplissant la zone de dispersion sont de forme quelconque. Ces éléments sont par exemple des billes et/ou des batonnets en matière céramique. Habituellement les éléments particulaires remplissant la zone de dispersion ont des dimensions de $10^{-5}$ à $3,5 \times 10^{-3}$ mètre, de préférence de $10^{-5}$ à $2 \times 10^{-3}$ mètre et avantageusement de $5.10^{-5}$ à $10^{-3}$ mètre.

Dans une forme préférée de réalisation du procédé selon la présente invention, le mélange gazeux issu de l'étape (a) est d'abord envoyé dans une zone de mélange en matière céramique définissant une multiplicité d'espaces présentant des passages entre les parois ayant, suivant au moins une direction, une dimension au plus égale à ladite distance de coincement de flamme, le mélange gazeux issu cette zone de mélange étant ensuite envoyé dans la zone de réaction, la distance inter-zones entre la zone de dispersion et la zone de mélange d'une part et entre la zone de mélange et la zone de réaction d'autre part étant au plus égale à ladite distance de coincement de flamme.

La dimension des passages entre les parois des éléments particulaires dans les zones de dispersion,de mélange et de réaction, correspondant à la distance de coincement de flamme est en général au plus égale à $10^{-2}$ m, avantageusement d'environ $5 \times 10^{-5}$ à $5 \times 10^{-3}$ m et de préférence d'environ $10^{-4}$ à $2 \times 10^{-3}$ m.

Il est préférable que les dimensions des passages entre les parois des éléments particulaires dans la zone

3

de dispersion et celles entre les parois des éléments particulaires ou des canaux du monolithe dans la zone de mélange soient inférieures à celles des passages entre les parois des éléments particulaires ou entre les parois des canaux du monolithe dans la zone de réaction. Ceci contribue à éviter sensiblement toute réaction d'oxydation dans les zones de dispersion et de mélange et au contraire à permettre à la réaction de se développer en phase gazeuse dans la zone de réaction, la réaction d'oxydation étant bien sûr contrôlée par le fait que les dimensions des passages dans la zone de réaction restent inférieures à la distance de coincement de flamme.

Il est également préférable pour les mêmes raisons que la distance entre la zone de dispersion et de mélange soit d'une part inférieure à celle entre les zones de mélange et de réaction et d'autre part inférieure à la dimension des passages des gaz dans la zone de réaction.

Les moyens d'accélération des gaz, noyés dans les éléments particulaires remplissant la zone de dispersion, employés dans le cadre de la présente invention peuvent être choisis parmi les moyens d'accélération de gaz bien connus de l'homme du métier. A titre d'exemple de moyen d'accélération des gaz on peut citer : les dispositifs convergent-divergent, par exemple ceux de type venturi, les éjecteurs, et les buses. Suivant les conditions opératoires et en particulier suivant la température des gaz introduits dans lesdits moyens d'accélération, ceux-ci peuvent comprendre un remplissage d'éléments particulaires permettant d'éviter tout risque d'explosion et d'effectuer le coincement de la flamme pouvant résulter de l'oxydation de ladite charge par le gaz oxydant. Le mode opératoire ci-dessus est particulièrement avantageux lorsque les deux gaz de la réaction sont introduits ensemble dans ces moyens d'accélération.

Par contre, lorsque les gaz sont introduits de manière séparée dans les accélérateurs, il n'est généralement pas utile de les remplir d'éléments particulaires. Ces accélérateurs peuvent alors fonctionner au maximum de leur possibilité.

Les moyens d'accélération des gaz utilisés dans la présente invention sont habituellement positionnés dans la zone de dispersion, à une distance telle que la sortie des gaz desdits moyens d'accélération se situe à une distance de la zone contiguë à la zone de dispersion (ou interface des zones) de 0,1 à 99,9 % de la valeur de la plus grande dimension de ladite zone de dispersion.

Dans une forme préférée de réalisation du procédé d'oxydation selon l'invention, on régule en continu la vitesse des gaz à la sortie des moyens d'accélération en jouant par exemple sur le débit et/ou la pression des gaz introduits dans lesdits moyens d'accélération de manière à ce que la température du mélange gazeux à l'entrée de la zone de réaction soit au plus égale à 800 °C et de préférence comprise entre 450 et 700 °C.

Dans les procédés d'oxydation de charges oxydables, il est préférable que la réaction d'oxydation ne démarre pas avant que le mélange charge oxydable - gaz oxydant ne soit bien fait c'est à dire avant l'obtention d'un mélange très homogène. Ainsi dans le procédé suivant la présente invention, les conditions sont de préférence choisies de manière à ce que la réaction d'oxydation ne démarre pas avant la zone de réaction. Ce but peut être atteint par exemple en choisissant une température des gaz à l'entrée de la zone d'accélération suffisamment basse et/ou l'obtention d'une vitesse suffisamment grande du mélange gazeux à la sortie des moyens d'accélération. Il peut être avantageux d'amorcer la réaction dans la zone de réaction, de préférence dans la partie de la zone de réaction contiguë à la zone de dispersion ou à la zone de mélange. Cet amorçage peut par exemple être réalisé par chauffage d'au moins une partie de la zone de réaction, par tout moyen connu de l'homme du métier, à une température au moins égale à 600 °C et de préférence de 700 à 1200 °C. Ce chauffage peut être effectué avant l'introduction de la charge oxydable et du gaz oxydant et/ou au moment du démarrage de cette introduction. Il n'est ni nécessaire, ni souhaitable de poursuivre ce chauffage après le démarrage de la réaction d'oxydation, celle-ci étant suffisamment exothermique pour se poursuivre sans apport de calories extérieures.

Le chauffage peut, par exemple, être réalisé par injection de gaz chauds provenant d'un brûleur, par oxydation d'au moins un hydrocarbure (par exemple hexane et/ou heptane) par un gaz contenant de l'oxygène ou par de l'oxygène essentiellement pur, cet hydrocarbure et l'oxygène étant introduits dans ladite partie de la zone de réaction.

L'amorçage peut également être effectué par d'autres moyens bien connus de l'homme du Métier par exemple à l'aide d'une étincelle produite par exemple à l'aide d'une bougie du type de celle que l'on utilise pour les moteurs à explosion, ou par un arc électrique produit, par exemple, entre deux électrodes.

Les éléments particulaires, la zone de mélange et la zone de réaction sont avantageusement en matière réfractaire céramique, qui peut être choisie par exemple dans le groupe comprenant la mullite, la cordiérite, les nitrures de silicium tels que $Si_3N_4$, les oxydes d'alcalino-terreux, les oxydes de métaux de transition et le carbure de silicium.

L'invention concerne aussi un réacteur d'oxydation d'une charge oxydable en phase gazeuse par un gaz ou un mélange de gaz comprenant au moins un gaz oxydant.

Le réacteur comporte des moyens d'alimentation en gaz oxydant et en charge oxydable et des moyens d'évacuation des produits réactionnels. Il comprend en outre, en combinaison, sur une partie au moins de sa section, des organes ou moyens de dispersion en matière céramique comportant un premier garnissage d'éléments particulaires en matière céramique ayant des dimensions telles que les espaces ou passages libres entre lesdits éléments aient, suivant au moins une direction, une dimension au plus égale à $10^{-2}$ mètre, lesdits éléments ayant habituellement des dimensions allant de $10^{-5}$ à $3,5 \times 10^{-3}$ mètre, dans lesquels est noyé au moins un moyen d'accélération des gaz relié aux moyens d'alimentation de la charge oxydable et du gaz oxydant, et sur une partie au moins de sa section, au moins un organe ou moyen de réaction, en matière céramique comportant un garnissage en matière céramique adapté à définir dans au moins une partie dudit moyen et avantageusement dans la totalité dudit moyen une multiplicité d'espaces présentant des passages ayant, suivant au moins une direction, une dimension au plus égale à $10^{-2}$ mètre, grâce auxquels les produits réactionnels sont canalisés vers lesdits moyens d'évacuation, la distance entre les moyens de dispersion et de réaction étant au plus égale à $10^{-2}$ mètre.

Dans le cas d'une accélération séparée de chaque gaz, la zone de dispersion comprendra au moins deux moyens d'accélération de la vitesse de ces gaz, chacun étant relié à un seul moyen d'alimentation, au moins un desdits moyens d'accélération étant relié au moyen d'alimentation en charge oxydable et au moins un desdits moyens d'accélération étant relié au moyen d'alimentation en gaz oxydant.

Selon un mode de réalisation préféré du réacteur, celui-ci comporte sur une partie au moins de sa section au moins un organe ou moyen de mélange, situé entre l'organe de dispersion et l'organe de réaction, définissant une multiplicité d'espaces présentant des passages ayant, suivant au moins une direction, une dimension au plus égale à $10^{-2}$ mètre, la distance entre l'organe de dispersion et l'organe de mélange d'une part et entre l'organe de mélange et l'organe de réaction d'autre part étant au plus égale à $10^{-2}$ mètre.

L'expression "dans au moins une partie" de l'organe de réaction, signifie au moins au voisinage immédiat de l'organe contigu à l'organe de réaction, c'est à dire au voisinage immédiat de l'organe de dispersion ou de l'organe de mélange.

Selon un premier mode de réalisation l'organe de mélange peut comporter une pluralité d'étages à mailles sensiblement décalées les unes par rapport aux autres.

Selon un deuxième mode de réalisation, l'organe de mélange peut comporter une pluralité de plaques, de préférence sensiblement verticales, comprenant de part et d'autre de chacune d'elles une pluralité de saillies et de canalisations inclinées de manière opposée de part et d'autre de chaque plaque, lesdites plaques étant disposées de telle façon que les saillies et les canalisations soient croisées.

Avantageusement les distances inter-organes sont au plus égales à $5 \times 10^{-3}$ mètre et de façon préférée de $10^{-4}$ à $2 \times 10^{-3}$ mètre.

Selon un mode premier de réalisation préféré, les espaces de l'organe de réaction sont définis par des garnissages en céramique qui peuvent comporter au moins un monolithe comprenant une pluralité de canaux juxtaposés sensiblement parallèles. De façon avantageuse, chacun de ces canaux unitaires a une section de $25 \times 10^{-10}$ à $10^{-4}$ mètre carré (m²) et de préférence de $10^{-8}$ à $25 \times 10^{-6}$ m².

Cette section est de forme quelconque, par exemple polygonale, circulaire ou elliptique, mais préférentiellement polygonale et par exemple triangulaire, carrée, rectangulaire ou hexagonale.

Lorsque l'organe de mélange comporte une pluralité d'étages à mailles décalées, les canaux du monolithe disposés dans les mailles de l'organe de mélange ont une section de préférence de $10^{-3}$ à $25 \times 10^{-6}$ m². De même, la section des canalisations, dans le cas des plaques disposées sensiblement dans le sens d'écoulement des gaz, mesurée une fois les plaques juxtaposées, est compatible avec la distance de coincement de flamme et donc de $25 \times 10^{-10}$ à $10^{-4}$ m² et de préférence de $10^{-3}$ à $25 \times 10^{-6}$ m². Cette section de canalisation peut être de forme quelconque, par exemple polygonale et de préférence carrée ou rectangulaire.

Les canalisations sont généralement inclinées de manière variable et le degré d'inclinaison peut varier de quelques degrés par rapport à la verticale à quelques degrés par rapport à l'horizontale. Il est de préférence compris entre 30 et 50 degrés.

Les plaques sont généralement de faible épaisseur pour améliorer le mélange. Elles sont généralement juxtaposées, mais elles peuvent éventuellement présenter un écartement tel qu'au niveau de l'intérieur des canalisations croisées, il reste compatible avec la distance de coincement de flamme.

Selon un deuxième mode de réalisation préféré, les espaces de l'organe de réaction sont définis par un garnissage comprenant des éléments particulaires, dont les dimensions sont avantageusement, dans le cas où il n'y a pas d'organe de mélange, sensiblement supérieures à celles des éléments particulaires remplissant l'organe de dispersion.

Dans le cas où il y a un organe de mélange intercalé entre l'organe de dispersion et l'organe de réaction, les éléments particulaires employés dans l'organe de réaction ont des dimensions de préférence supérieures à celle des éléments particulaires remplissant l'organe de dispersion et à celles des éléments particulaires ou des canaux de monolithe dans l'organe de mélange.

Lesdits éléments particulaires de la zone de réaction, ont habituellement des dimensions de $10^{-5}$ à $4 \times 10^{-3}$ m, de préférence de $1,5 \times 10^{-5}$ à $2,5 \times 10^{-3}$ m, et le plus souvent de $5 \times 10^{-5}$ à $1,5 \times 10^{-3}$m.

Dans un mode de réalisation particulièrement avantageux, le réacteur comprend un organe de dispersion, un organe de mélange et un organe de réaction, ledit organe de mélange tel que défini ci-avant comporte en outre sur une partie au moins un remplissage d'éléments particulaires de dimensions sensiblement inférieures à celles d'un canal unitaire du monolithe ou de la canalisation (dans des plaques juxtaposées) et ledit organe de réaction comporte au moins un monolithe au moins en partie rempli d'éléments particulaires de dimensions sensiblement inférieures à celles d'un canal unitaire, lesdits éléments particulaires étant habituellement retenus au niveau des divers organes par des grilles en matériau céramique.

Si la dimension de chaque canal unitaire dans l'organe de mélange et/ou dans l'organe de réaction est compatible avec la distance de coincement de flamme, on peut concevoir de ne remplir d'éléments particulaires qu'une partie de ces organes ou qu'une partie de l'un d'entre eux seulement.

On peut ainsi par exemple ne remplir, partiellement ou en totalité, d'éléments particulaires que le monolithe de l'organe de réaction ou que le monolithe de l'organe de mélange ou bien les deux.

Le garnissage de l'organe de réaction peut également, selon un autre mode de réalisation comprendre au moins un élément catalytique et/ou au moins un catalyseur seuls ou combinés avec les éléments particulaires décrits ci-avant. Ce catalyseur peut être supporté soit par les parois du monolithe soit par les éléments particulaires. Le catalyseur peut comprendre un support, par exemple de l'alumine ou de la silice sur lequel on a déposé, par exemple, du chlorure de cuivre et du chlorure de potassium, de l'oxyde de vanadium éventuellement associé à du sulfate de potassium, du cérium, du lanthane, ou un composé de cérium ou de lanthane, du phosphomolybdate de bismuth, ou du molybdate de cobalt. Le catalyseur peut également comprendre des oxydes métalliques tels que des oxydes d'argent et/ou de cuivre et du carbure de silicium poreux recouvert d'argent.

Les éléments particulaires décrits ci-avant permettent de diminuer les dimensions de coincement de flamme à une valeur qu'il est difficile d'atteindre par la technologie sans risque de malfaçon et par conséquent de réaliser des réactions d'oxydation en présence d'oxygène sensiblement pur.

L'organe de mélange peut comprendre un nombre N d'étages de mélange dûs à la présence, par exemple, de N disques, habituellement de faible épaisseur, à mailles décalées ou décentrées permettant de parfaire le mélange des gaz par division de l'écoulement.

Le nombre de disques est habituellement de 3 à 50 et de préférence de 6 à 20. L'épaisseur unitaire des disques est habituellement de $10^{-3}$ à $10^{-2}$ m et de préférence d'environ $5.10^{-3}$ m. Les disques sont habituellement disposés dans un plan sensiblement perpendiculaire à l'écoulement des gaz, mais ils peuvent être orientés différemment. Les disques sont habituellement juxtaposés mais ils peuvent éventuellement présenter un écartement tel que les espaces ainsi définis restent compatibles avec la distance de coincement de flamme.

La zone réactionnelle proprement dite permet l'avancement de la réaction d'oxydation et son contrôle compte tenu des niveaux thermiques atteints et l'évacuation des produits réactionnels.

En raison des faibles dimensions des espaces dans l'organe de dispersion et dans l'organe de réaction, de la faible section de mailles ou des canalisations de l'organe de mélange, de la faible section des canaux de l'organe de réaction dans le cas où il comprend au moins un monolithe et en raison également de la faible distance entre les divers organes dans le réacteur, on réalise un procédé d'oxydation et un réacteur permettant sa mise en oeuvre, sans qu'il y ait de rétromélange, ni d'explosion ou retour de flamme.

La longueur des divers organes qui composent le réacteur est habituellement de $10^{-2}$ à 3 mètres. La longueur de la zone de réaction représente en général de 50 à 90 % de la longueur totale du réacteur et la zone de mélange, par exemple de 50 à 70 % de la longueur disponible pour la zone de dispersion et de mélange.

L'ensemble des éléments en matériau céramique réfractaire est de mise en oeuvre aisée, neutre vis à vis de la charge, du gaz oxydant et des produits réactionnels et le réacteur peut ainsi fonctionner à des températures de parois atteignant 1200 à 1500 °C ou même plus.

On obtient des résultats particulièrement intéressants, sans dépot de coke par exemple et avec une excellente conversion du méthane et une proportion de dioxyde de carbone dans les produits de réaction relativement faible, lorsque les surfaces des sections des divers organes contigus sont sensiblement égales. De façon très avantageuse les divers garnissages introduits dans le réacteur occupent toute la surface de la section du réacteur qui est par exemple circulaire lorsque le réacteur est tubulaire. Il est également possible que les monolithes utilisés aient une section polygonale par exemple carrée ou rectangulaire ou tout autre forme et il en est de même pour la section de l'organe de dispersion.

L'obtention de mélange particulièrement homogène à l'entrée de la zone de réaction permet d'obtenir d'excellentes performances. Les principaux facteurs qui permettent d'obtenir un mélange très homogène sont :

1 - la vitesse des gaz à la sortie des moyens d'accélération et

2 - la position des moyens d'accélération dans l'organe de dispersion.

Ainsi dans le cas où le réacteur ne comprend qu'un organe de dispersion et un organe de réaction il est préférable d'opérer avec des vitesses des gaz, à la sortie de moyens d'accélération, relativement importantes c'est à dire ayant des valeurs élevées tout en restant dans l'intervalle de vitesses défini ci-avant.

De même dans ce cas, les moyens d'accélération seront disposés de manière à ce que la sortie des gaz, desdits moyens d'accélération, se situe à une distance relativement grande de l'organe de réaction, ladite distance étant par exemple égale à 20 à 99,9 % et de préférence à 40 à 99,9 % de la valeur de la plus grande dimension de l'organe de dispersion.

Dans le mode de réalisation préféré de l'invention dans lequel le réacteur comprend un organe de dispersion, un organe de mélange et un organe de réaction, il est possible d'opérer avec des vitesses de gaz, à la sortie des moyens d'accélération, ayant des valeurs relativement modérées tout en restant à l'intérieur de l'intervalle de vitesses défini ci-avant. De même, les moyens d'accélération seront alors disposés de manière à ce que la sortie des gaz desdits moyens d'accélération se situe à une distance relativement faible de l'organe de mélange, ladite distance étant par exemple égale à 0,1 à 80 % et, de préférence, 0,1 à 50 % de la valeur de la plus grande dimension dudit organe de dispersion. De plus, dans ce cas, en fonction de la vitesse des gaz et de la position des moyens d'accélération, le nombre d'étages de l'organe de mélange pourra être plus ou moins grand. A titre d'exemple,ce nombre pourra être relativement faible, par exemple de 3 à 15 lorsque la vitesse est relativement grande, par exemple environ 50 à 150 m x s$^{-1}$ et/ou lorsque la sortie des gaz desdits moyens d'accélération est relativement éloignée de l'organe de mélange, par exemple environ à une distance correspondant à 30 à 50 % de la valeur de la plus grande dimension de l'organe de dispersion.

Selon un mode de réalisation avantageux, le réacteur comprend au moins un moyen de mesure et de régulation de la température situé au voisinage immédiat de l'extrémité de l'organe de réaction, du côté où arrive le mélange gazeux, ledit moyen coopérant avec les moyens d'introduction ou d'alimentation des gaz c'est à dire de la charge oxydable et du gaz oxydant de manière à réguler la vitesse des gaz à la sortie des moyens d'accélération et à maintenir la température à l'entrée de l'organe de réaction en dessous d'une valeur prédéterminée.

Le réacteur peut également comprendre de façon avantageuse des moyens d'amorçage de la réaction situés dans l'organe de réaction et de préférence, à proximité de l'extrémité de l'organe de réaction du côté où arrive le mélange gazeux et en aval des moyens de mesure de la température. Lesdits moyens d'amorçage de la réaction sont choisis parmi l'un de ceux permettant de réaliser l'amorçage de la réaction tels que décrits ci-avant. Ces moyens d'amorçage seront par exemple des moyens de chauffage d'au moins une partie de l'organe de réaction.

La charge oxydable utilisable dans le cadre de l'invention comprend, par exemple, des hydrocarbures aliphatiques saturés tel que le méthane et les effluents du procédé de reformage à la vapeur, l'orthoxylène, le naphtalène, le benzène, le méthanol, le mélange méthane-toluène et le mélange éthylène-acide chlorhydrique.

L'invention sera mieux comprise par la description de quelques modes de réalisation, donnés à titre illustratif, mais nullement limitatif, qui en sera faite ci-après à l'aide des figures annexées :

Les figures 1A et 1B représentent schématiquement deux modes de réalisation du procédé de l'invention suivant une coupe longitudinale.

Les figures 2A et 2B représentent schématiquement deux modes préférés de réalisation du procédé suivant l'invention .

Les figures 3A, 3B, 3C et 3D montrent une vue schématique de dessus, des disques de l'organe de mélange selon trois modes de réalisation différents.

La figure 3E montre le cheminement des gaz dans l'organe de dispersion et dans l'organe de mélange.

La figure 4 illustre les plaques utilisées dans l'organe de mélange.

La figure 5 représente une vue schématique du monolithe de l'organe de réaction.

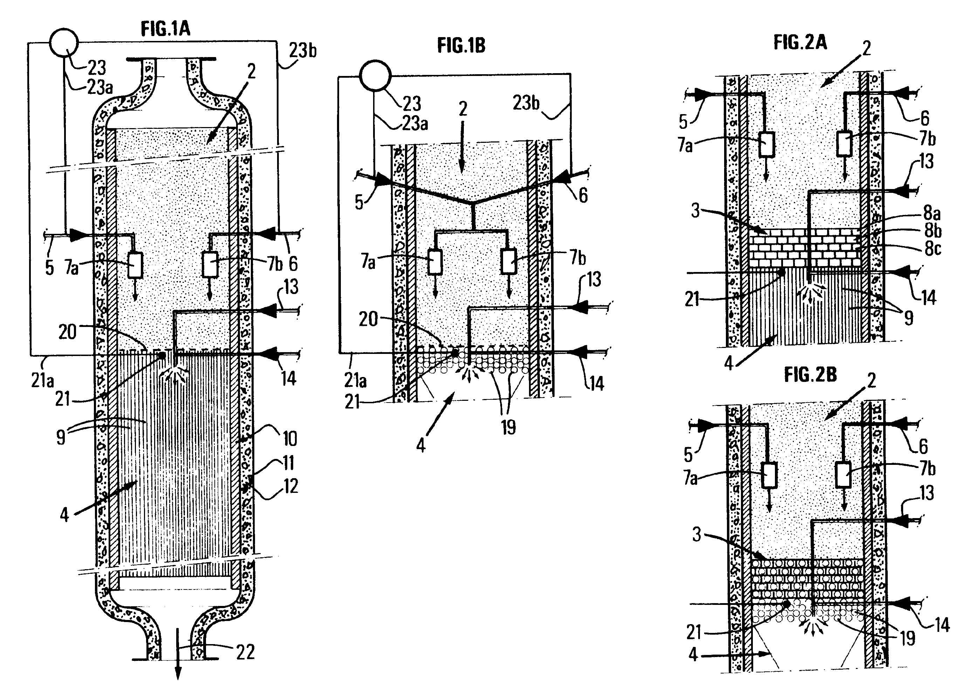

Sur les figures 1A et 1B, on a représenté, selon un mode de réalisation, un réacteur d'oxydation (1) vertical, cylindrique, de forme allongée comprenant un premier garnissage d'éléments particulaires, décrits ci-avant, dans la zone de dispersion (2), une grille (20) retenant les éléments particulaires, et un monolithe, en mullite, de forme cylindrique mais dont la section utilisée est, par exemple, carrée dans lequel sont ménagés des canaux (9), sensiblement parallèles entre eux et à l'axe du réacteur, ledit monolithe formant la zone de réaction (4) (figure 1A et 5). Dans la figure 1B, la zone de réaction (4) comprend un second garnissage d'éléments particulaires (19). Dans le mode de réalisation représenté sur la figure 1A, le monolithe en mullite représentant la zone réactionnelle (figure 5) est de forme cylindrique mais de section utilisée semblable à celle du premier garnissage d'éléments particulaires de la zone de dispersion (2). Il comporte une pluralité de canaux (9) juxtaposés, sensiblement parallèles entre eux et à l'axe du réacteur. La section individuelle de chaque canal (9), de forme carrée par exemple, est de 1 x 10$^{-6}$ m$^2$ environ et leur longueur est, par exemple, d'environ 0,5 m.

Ces canaux (9) sont d'une part destinés à canaliser les produits de réaction jusqu'à une ligne d'évacuation (22) et d'autre part, en raison de leur faible dimension et par effet de paroi, à assurer le coincement de la flamme,

7

ce qui permet à la réaction de s'effectuer sans explosion.

Dans le mode de réalisation représenté sur la figure 1B, la zone réactionnelle (4) comprend ledit second garnissage d'éléments particulaires, par exemple des billes (19) de dimension choisies en fonction de la distance de coincement de flamme et qui sont retenues par une grille (20) ou un catalyseur. Ce mode de réalisation est en particulier préférable lorsque l'on effectue des réactions d'oxydation sous pression dans des conditions voisines de la stoéchiométrie et/ou lorsqu'on opère avec un gaz très riche en oxygène, nécessitant des distances de coincement de flamme très faibles, ce qui est difficilement réalisable sur le plan technologique dans le cas du mode de réalisation selon la figure 1A.

Dans l'exemple de réalisation selon la figure 1A, la charge oxydable préalablement chauffée, par exemple à une température d'environ 600 à 800 °C, arrive par le conduit (6) dans un moyen d'accélération (7b) noyé dans le premier garnissage et le gaz oxydant préchauffé par exemple à une température d'environ 50 à 250 °C arrive par le conduit (5) dans un autre moyen d'accélération (7a) noyé dans ce même garnissage, ces moyens (7a) et (7b) étant disposés de manière sensiblement symétrique par rapport à l'axe du réacteur.

La sortie des gaz des accélérateurs (7a) et (7b) se situe à une distance de l'extrémité la plus proche de la zone de réaction égale à environ 50 % de la longueur totale de la zone de dispersion.

Selon la figure 1B on a représenté un autre mode de réalisation de l'invention où la charge oxydable et le gaz oxydant alimentent ensemble chaque accélérateur (7) respectivement par les lignes (6) et (5).

La réaction d'oxydation est initiée par préchauffage de la zone de réaction avant et/ou pendant le début de l'introduction des gaz par les lignes (5) et (6), par introduction d'oxygène par le conduit (14) et d'heptane par le conduit (13) de manière à porter la partie supérieure de la zone de réaction à une température permettant l'amorçage de la réaction par exemple environ 600 à 700 °C. On dispose un moyen (21) de mesure de la température à l'interface de la zone de dispersion et de la zone de réaction. Ce moyen coopère par une ligne (21a) avec un moyen de régulation (23) des débits d'alimentation en charge oxydable et en gaz oxydant relié aux moyens d'alimentation (5) et (6) par les lignes (23a) et (23b), de manière à ce que la température qu'il mesure, reste comprise, par exemple, entre 600 et 800 °C, bornes incluses, durant tout le temps de fonctionnement du réacteur.

Le réacteur comprend par exemple sur toute sa longueur un manchon (10) en mullite, un manchon en béton réfractaire (12) et une paroi externe en acier (11) l'ensemble permettant de réaliser des réactions sous pressions élevées et à très haute température.

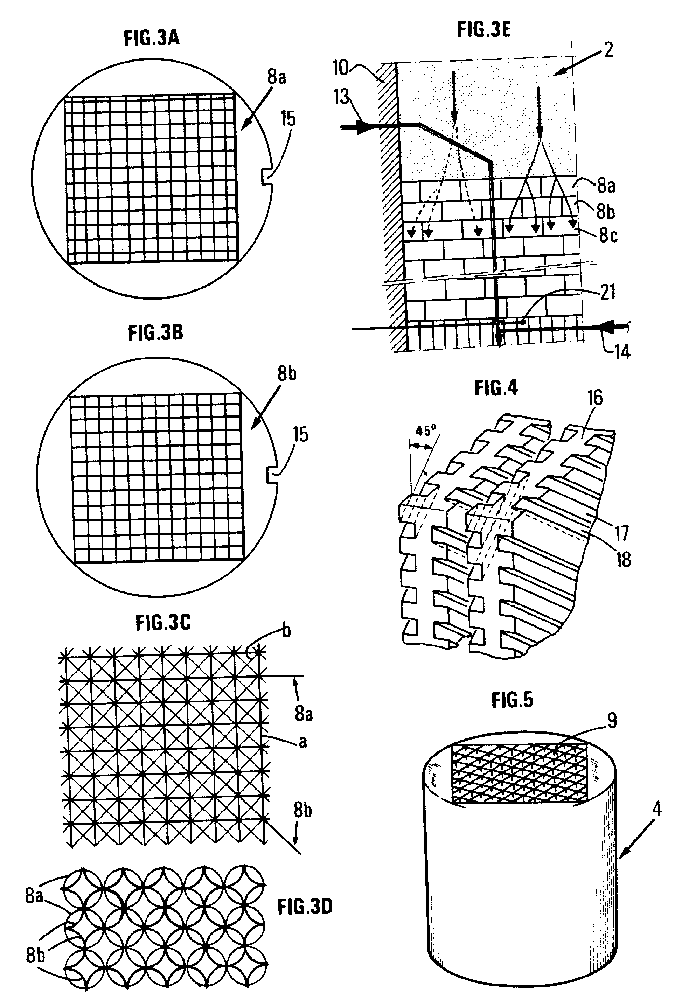

Les figures 2A et 2B représentent un mode de réalisation préféré de l'invention dans lequel le réacteur d'oxydation (1) comprend une zone de mélange (3), par exemple en mullite constituée par une pluralité de disques (8a), (8b), (8c), de forme cylindrique, de faible épaisseur et à mailles décalées (figures 3A, 3B).

Les autres éléments formant le réacteur sont identiques à ceux décrits en liaison avec la description des figures 1A et 1B et portent les mêmes numéros de référence. Sur la figure 2B, le mélangeur et la zone de réaction comprennent un remplissage d'éléments particulaires, par exemple des billes(19). Il est également possible de réaliser un réacteur comprenant un organe de dispersion (2) comportant un premier garnissage d'éléments particulaires, un organe de mélange (3) ne comportant pas de remplissage d'éléments particulaires et un organe de réaction (4) comportant un second garnissage d'éléments particulaires.

La section de chaque maille ou canal unitaire de monolithe au niveau de chaque disque (8) du mélangeur (3) est telle qu'elle correspond, dans le cas où il n'y a pas de remplissage par des éléments particulaires, à une dimension au plus égale à la distance de coincement de flamme.

La distance entre les divers organes est au plus égale à $10^{-2}$ m et de préférence de $10^{-4}$ à $2 \times 10^{-3}$ m.

Avantageusement, les surfaces en vis à vis des diverses zones sont sensiblement égales. Ces surfaces sont déterminées par un plan perpendiculaire à la direction de l'écoulement des gaz ou des canaux du ou des monolithes. De façon préférée elles sont sensiblement égales à la section utile du réacteur par un plan perpendiculaire à son axe, de sorte que toute la surface utile du réacteur est utilisée à pleine capacité.

Les figures 3A, 3B, 3C et 3D représentent des modes de réalisation différents du mélangeur (3). Celui-ci comprend une pluralité de disques (8a), (8b), (8c) cylindriques, de diamètre de préférence égal à celui du réacteur et d'épaisseur comprise entre $10^{-3}$ et $10^{-2}$ m. Chaque disque (8) est garni d'un maillage de canaux (monolithe) dont la section a été définie ci-avant et qui correspond à la surface d'un carré dont l'une au moins des dimensions est au plus égale à la distance de coincement de flamme.

Le maillage de chaque disque (figures 3A et 3B) est décalée selon deux directions perpendiculaires x et y du plan, de préférence de a/2 si a est le côté du carré.

Les disques sont empilés et maintenus en place par exemple par une barette, non représentée sur les figures, qui se loge dans une encoche (15) prévue à cet effet. On aboutit ainsi à un cheminement des gaz représenté sur la figure 3E qui permet de parfaire le mélange des gaz et d'obtenir un mélange très homogène à la sortie de l'organe de mélange.

Selon un autre mode de réalisation représenté par la figure 3C, les disques (8a), (8b), formés par un mono-

EP 0 334 710 B1

lithe comportant des canaux par exemple de section carrée, peuvent être disposés en alternance de sorte que l'un des disques ait ses mailles ou canaux orientés suivant la diagonale des mailles ou canaux de l'autre disque et ainsi de suite.

On peut avantageusement, par exemple, réaliser l'empilement des disques en alternance à 45 °C avec des tailles de mailles "a" pour un disque et une taille de maille "b = a √2/2" pour l'autre disposé à 45 °.

Les canaux selon la figue 3D peuvent aussi être ronds et les canaux d'un disque peuvent être décalés par exemple suivant un seul axe du plan ou suivant deux de ses axes (figure 3D).

La figure 4 représente un autre exemple de réalisation de l'organe de mélange. Il comprend une pluralité de plaques (16) disposées habituellement sensiblement selon l'axe d'écoulement des gaz et avantageusement de façon sensiblement verticale et sensiblement parallèle à l'axe des canaux (9) dans le cas où la zone de réaction comprend un monolithe.

Chaque plaque, dont les dimensions sont par exemple $(2.10^{-1})$ x $(5.10^{-2})$ x $(3.10^{-3})$ m, est creusée de part et d'autre de canalisations (17) d'environ $10^{-3}$ m de largeur et $10^{-3}$ m de profondeur.

Les saillies (18) ainsi déterminées ont sensiblement les mêmes dimensions. Les canalisations (17) sont inclinées d'environ 45 ° par rapport à l'axe du réacteur ou à l'écoulement des gaz sur une de ses faces et sensiblement du même angle, mais en direction opposée sur l'autre face. Elles peuvent être juxtaposées de façon que les faces en contact de deux plaques adjacentes présentent des canalisations croisées, ce qui favorise efficacement le mélange des fluides.

Dans l'exemple représenté sur la figure 4, on a cependant écarté les deux plaques pour mieux illustrer le croisement des canalisations (17).

A la sortie du mélangeur, la réaction d'oxydation est initiée et se poursuit tout au long de la zone de réaction.

Grâce à ces dispositions et aux matériaux utilisés, on parvient à réaliser des réactions d'oxydation à des températures très élevées de l'ordre de 1.300 °C par exemple, sans dépôt inopportun de carbone et avec des temps de séjour dans le réacteur n'excédant pas, par exemple, 1 s, tout en protégeant le réacteur de la chaleur dégagée lors de la réaction.

Par ailleurs les diverses dispositions selon l'invention permettent d'utiliser le gaz oxydant à une température relativement basse et par exemple plus basse que lorsque le contact entre le gaz oxydant et la charge oxydable ne se produit qu'après un certain délai, comme cela est par exemple, le cas dans le procédé selon la demande de brevet EP-A-231706.

L'échange de chaleur entre la charge oxydable et le gaz oxydant est en effet plus efficace dans le procédé selon l'invention que dans le procédé EP-A-231706 et permet d'obtenir une température du mélange des gaz ayant la valeur désirée tout en ayant une faible température du gaz oxydant. Ceci est en particulier très avantageux dans le cas de l'utilisation de l'oxygène sensiblement pur dont l'effet corrosif à haute température pose un certain nombre de problèmes difficiles à résoudre.

Les exemples suivants illustrent l'invention sans en limiter la portée.

EXEMPLES 1 ET 2

On réalise un réacteur (1) vertical de forme tubulaire comportant :
– un premier garnissage (2) de billes en carbure de silicium de diamètre moyen 25 x $10^{-5}$ m, ce qui détermine un passage entre les parois de ces billes de 25 x $10^{-5}$ m. Ce garnissage a une hauteur de 18 x $10^{-2}$ m et une section circulaire de 4 x $10^{-2}$ m.

A l'intérieur de ce garnissage et dans un plan sensiblement perpendiculaire à l'axe du réacteur, on dispose cinq dispositifs convergent-divergent ; quatre (7b) sont répartis suivant les sommets d'un carré sensiblement de même axe de symétrie que l'axe du réacteur et sont adaptés à recevoir la charge oxydable par la ligne (6) tandis que le cinquième (7a) au centre du carré reçoit par la ligne (5) une alimentation en oxygène de sorte que les sorties des gaz de ces dispositifs se trouvent à 3 x $10^{-2}$ m du mélangeur (3).
Les billes sont retenues par une grille en mullite.
– Un mélangeur (3), dont la section a la même surface que celle du garnissage (2), formé de monolithes en mullite de 5 x $10^{-3}$ m d'épaisseur dont la section de la maille unitaire a une surface de 64 x $10^{-8}$ m². Le premier de ces monolithes est plaqué contre la grille qui retient les billes du premier garnissage. On alterne, comme décrit ci-avant, sept monolithes de hauteur totale 3,5 x $10^{-2}$ m dont le centre correspond au croisement de deux parois de canaux du monolithe (4) formant la zone de réaction et sept monolithes de même hauteur totale dont le centre correspond au centre d'un canal du monolithe de la zone de réaction. Les monolithes sont remplis de billes en carbure de silicium de diamètre moyen 25 x $10^{-5}$ m.
Les billes sont retenues par une grille en mullite.
– Un monolithe (4) en mullite, dont la section de chaque canal a une surface de 64 x $10^{-8}$ m², d'une longueur de 45 x $10^{-2}$ m et dont la section a la même surface que celle du mélangeur (3). Ce monolithe est en contact

9

avec le mélangeur.

L'ensemble du réacteur (2), (3) et (4) est tenu par un manchon en mullite de 0,7 m de longueur.

La charge oxydable est introduite à 757 °C dans les dispositifs d'accélération (7b). Sa répartition molaire est la suivante :

|                    |        |
|--------------------|--------|
| méthane            | 25,55  |
| hydrogène          | 42,74  |
| dioxyde de carbone | 8,18   |
| monoxyde de carbone| 6,09   |
| eau                | 46,76. |

L'oxygène sensiblement pur est introduit à 150 °C par les moyens (7a) à raison de 0,52 mole d'oxygène par mole de méthane.

EXEMPLE 1 (comparatif)

On introduit les gaz par les lignes (5) et (6) à un débit tel que leur pression en sortie des dispositifs convergent-divergent soit égale à 4 MPa et leur température soit égale à 680 °C environ.

La somme des débits de la charge oxydable et de l'oxygène est de $0,887 \times 10^{-3}$ $m^3 \times s^{-1}$. La vitesse des gaz mesurée à la sortie des dispositifs d'accélération (7) est égale à 5 m x $s^{-1}$.

En sortie de réacteur la température est de 972,5 °C, le produit de réaction a la composition molaire donnée dans le tableau 1 ci-après.

EXEMPLE 2

On répète l'exemple 1 la pression en sortie des dispositifs convergent-divergents est fixée à 4 MPa et la somme des débits à 4,435 $m^3 \times s^{-1}$. La vitesse des gaz mesurée à la sortie des dispositifs d'accélération (7) est égale à 25 m x $s^{-1}$. En sortie de réacteur la température est de 971,7 °C, le produit de réaction a la composition molaire donnée dans le tableau 1 ci-après.

EXEMPLE 3

On réalise un réacteur (1) vertical, de forme tubulaire comprenant :

– un premier garnissage (2) de $25 \times 10^{-2}$ m de longueur, de billes ayant les caractéristiques de celui défini dans les exemples 1 et 2. A l'intérieur du garnissage, on dispose cinq dispositifs convergent-divergent selon la configuration décrite dans les exemples 1 et 2. Les sorties des gaz de ces dispositifs sont situées à une distance de $12 \times 10^{-2}$ m de l'extrémité du garnissage contigu au monolithe (4) où s'effectue la réaction. Les billes sont retenues par une grille en mullite,

– un monolithe (4) en mullite semblable à celui des exemples 1 et 2, de $45 \times 10^{-2}$ m de longueur et de même surface que celle du garnissage (2). Ce monolithe est en contact avec la grille.

L'ensemble du réacteur est tenu par un manchon en mullite de $70 \times 10^{-2}$ m de longueur.

La charge et le gaz oxydant sont identiques à ceux introduits selon l'exemple 1 et ils sont introduits dans les conditions suivantes :

– pression égale à 4 MPa en sortie des dispositifs convergent-divergents

– somme des débits égale à 17,74 $m^3 \times s^{-1}$

– vitesse des gaz mesurée à la sortie desdits dispositifs égale à 100 m x $s^{-1}$

– température des gaz en sortie de réacteur : 971,5 °C.

Le produit de réaction a la composition molaire donnée dans le tableau 1, exemple 3, ci-après.

EP 0 334 710 B1

TABLEAU 1

| EXEMPLE | 1 | 2 | 3 |
|---|---|---|---|
| Méthane | 3,40 | 3,76 | 3,96 |
| Hydrogène | 83,44 | 83,14 | 82,82 |
| Dioxyde de carbone | 9,87 | 10,01 | 10,10 |
| Monoxyde de carbone | 25,52 | 25,55 | 25,56 |
| Eau | 50,36 | 50,04 | 49,85 |
| Suies * | 0,558 | 0,275 | 0,114 |

\* en gramme par mole de méthane converti

**Revendications**

1. Procédé d'oxydation, d'une charge oxydable en phase gazeuse par un gaz ou un mélange de gaz comprenant au moins un gaz oxydant caractérisé en ce qu'il comprend les étapes suivantes :

a) on introduit la charge oxydable et le gaz oxydant, dans une zone de dispersion entièrement remplie d'éléments particulaires en matière céramique ayant des dimensions telles que les espaces ou passages libres entre lesdits élements aient, suivant au moins une direction, une dimension au plus égale à la distance de coincement de la flamme pouvant résulter de l'oxydation de ladite charge par ledit gaz oxydant, ladite charge et ledit gaz étant introduits dans ladite zone de dispersion à l'aide d'au moins un moyen d'accélération des gaz, noyé dans lesdits éléments particulaires, permettant d'obtenir une vitesse des gaz à la sortie dudit moyen d'accélération d'au moins 10 m x s$^{-1}$,

b) on fait réagir le mélange gazeux issu de l'étape (a) dans une zone de réaction en matière céramique comprenant une multiplicité d'espaces présentant des passages ayant, suivant au moins une direction, une dimension au plus égale à ladite distance de coincement de flamme, la distance inter-zones étant au plus égale à ladite distance de coincement de flamme.

2. Procédé selon la revendication 1, dans lequel la dimension des passages dans la zone de réaction est supérieure à celle des passages dans la zone de dispersion.

3. Procédé selon la revendication 1 ou 2 dans lequel le mélange gazeux issu de l'étape (a) est envoyé dans une zone de mélange en matière céramique définissant une multiplicité d'espaces présentant des passages ayant, suivant au moins une direction, une dimension au plus égale à ladite distance de coincement de flamme, le mélange gazeux issu de cette zone de mélange étant ensuite envoyé dans la zone de réaction, la distance inter-zones entre la zone de dispersion et la zone de mélange d'une part et entre la zone de mélange et la zone de réaction d'autre part, étant au plus égale à ladite distance de coincement de flamme.

4. Procédé selon la revendication 3 dans lequel la dimension des passages dans la zone de réaction est d'une part supérieure à la dimension des passages dans la zone de mélange et dans la zone de dispersion et d'autre part supérieure à la distance entre la zone de dispersion et la zone de mélange.

5. Procédé selon l'une des revendications 1 à 4 dans lequel ladite zone de dispersion est entièrement remplie d'éléments particulaires en matière céramique ayant des dimensions de $10^{-5}$ à $3,5 \times 10^{-3}$ mètre.

6. Procédé selon l'une des revendications 1 à 5 dans lequel la sortie des gaz desdits moyens d'accélération est située à une distance de la zone contiguë à la zone de dispersion de 0,1 % à 99,9 % de la valeur de la plus grande dimension de ladite zone de dispersion.

7. Procédé selon l'une des revendications 1 à 6 dans lequel la vitesse des gaz à la sortie desdits moyens d'accélération est régulée en continu de manière à ce que la température du mélange gazeux à l'entrée de la zone de réaction soit au plus égale à 800 °C.

8. Procédé selon l'une des revendications 1 à 6 dans lequel la réaction d'oxydation est amorcée par chauffage à une température de 700 à 1200 °C d'au moins une partie de la zone de réaction contigüe à la zone de dispersion ou à la zone de mélange.

9. Réacteur d'oxydation pour la mise en oeuvre du procédé selon la revendication 1, comportant des moyens d'alimentation en gaz oxydant (5), des moyens d'alimentation en charge oxydable (6), des moyens d'évacuation des produits réactionnels (13), et, sur au moins une partie de la section dudit réacteur, au moins un moyen de réaction (4) en matière céramique connecté auxdits moyens d'évacuation et comportant un garnissage en matière céramique adapté à définir dans au moins une partie dudit moyen une multiplicité d'espaces présentant des passages (9) ayant, suivant au moins une direction, une dimension au plus égale à $10^{-2}$ mètre, ledit réacteur étant caractérisé en ce qu'il comprend, sur au moins une partie de sa section, des moyens de dispersion (2) en matière céramique comportant un premier garnissage d'éléments particulaires en matière céramique ayant des dimensions telles que les passages libres entre lesdits éléments aient, suivant au moins une direction, une dimension au plus égale à $10^{-2}$ m, lesdits éléments ayant des dimensions de $10^{-5}$ à $3,5 \times 10^{-3}$ mètre dans lesquels :
   – soit est noyé au moins un moyen d'accélération (7a) relié aux moyens d'alimentation en gaz oxydant (5) et en charge oxydable (6),
   – soit sont noyés au moins deux moyens d'accélération (7a et 7b), chacun étant relié à un seul moyen d'alimentation, au moins un desdits moyens d'accélération (7b) étant relié au moyen d'alimentation en charge oxydable (6) et au moins un desdits moyens d'accélération (7a) étant relié au moyen d'alimentation en gaz oxydant (5), la distance entre lesdits moyens de dispersion (2) et ledit moyen de réaction (4) étant au plus égale à $10^{-2}$ m.

10. Réacteur selon la revendication 9, caractérisé en ce qu'il comprend au moins un moyen de mélange (3) en matière céramique situé entre les moyens de dispersion et les moyens de réaction, comportant une pluralité d'étages (8a, 8b, 8c) à mailles sensiblement décalées, chacune de ces mailles définissant sur toute la longueur dudit moyen de mélange (3) une multiplicité d'espaces présentant des passages ayant, suivant au moins une direction, une dimension au plus égale à $10^{-2}$ mètre et la distance entre les moyens de dispersion et le moyen de mélange d'une part et entre le moyen de mélange et les moyens de réaction d'autre part étant au plus égale à $10^{-2}$ mètre.

11. Réacteur selon la revendication 9, caractérisé en ce qu'il comprend un moyen de mélange (3) en matière céramique situé entre les moyens de dispersion et les moyens de réaction, comportant une pluralité de plaques (16) sensiblement verticales comprenant de part et d'autre de chacune d'elles une pluralité de saillies (17) et de canalisation (18) inclinées de manière opposée de part et d'autre de chaque plaque, lesdites plaques étant disposées de telle façon que les saillies (17) et les canalisations (18) soient croisées, la multiplicité d'espaces définis par lesdites plaques présentant des passages ayant, suivant au moins une direction une dimension au plus égale à $10^{-2}$ mètre et la distance entre les moyens de dispersion et le moyen de mélange d'une part et entre le moyen de mélange et les moyens de réaction d'autre part étant au plus égale à $10^{-2}$ mètre.

12. Réacteur suivant l'une des revendications 9 à 11, caractérisé en ce que le garnissage des moyens de réaction (4) comporte au moins un monolithe en céramique comprenant une pluralité de canaux juxtaposés.

13. Réacteur suivant la revendication 12, caractérisé en ce que le monolithe est au moins en partie rempli d'éléments particulaires en céramique.

14. Réacteur suivant l'une des revendications 9 à 11, caractérisé en ce que le garnissage des moyens de réaction (4) comprend des éléments particulaires en céramique ayant des dimensions de $10^{-5}$ à $4 \times 10^{-3}$ mètre, les dimensions desdits éléments particulaires dans lesdits moyens de réaction étant supérieures à celles des éléments particulaires remplissant lesdits moyens de dispersion (2).

15. Réacteur suivant l'une des revendications 9 à 14, caractérisé en ce que le garnissage desdits moyens de réaction comprend dans une partie au moins desdits moyens au moins un catalyseur.

16. Réacteur suivant l'une des revendications 9 à 15, caractérisé en ce qu'il comprend des moyens (21) de mesure de la température situés au voisinage immédiat de l'extrémité des moyens de réaction du côté où arrive le mélange gazeux et des moyens de régulation de débit (23) reliés auxdits moyens d'alimentation (5 et 6), lesdits moyens de mesure coopérant avec les moyens de régulation des débits d'alimentation en gaz de manière à réguler la vitesse des gaz à la sortie des moyens d'accélération et à maintenir la température à l'entrée des moyens de réaction en dessous d'une valeur prédéterminée.

17. Réacteur selon l'une des revendications 9 à 16, caractérisé en ce qu'il comprend des moyens de chauffage d'au moins une partie des moyens de réaction

18. Utilisation du réacteur selon l'une des revendications 9 à 17 notamment pour la synthèse de méthanol et alcools homologues supérieurs à partir d'oxydes de carbone et d'hydrogène.

**Patentansprüche**

1. Verfahren zur Oxidation einer oxidierbaren Charge in der Gasphase durch ein Gas oder ein Gasgemisch, das mindestens ein oxidierendes Gas enthält, dadurch gekennzeichnet, daß es die folgenden Stufen umfaßt:

(a) man führt die oxidierbare Charge und das oxidierende Gas in eine Dispersionszone ein, die vollständig gefüllt ist mit teilchenförmigen Elementen aus einem Keramikmaterial mit solchen Dimensionen, daß die Hohlräume (Zwischenräume) oder freien Durchgänge zwischen diesen Elementen wenigstens in einer Richtung eine Dimension haben, die höchstens gleich dem Hemmungsabstand von der Flamme ist, die aus der Oxidation der genannten Charge durch das genannte oxidierende Gas resultieren kann, wobei die Charge und das Gas in diese Dispersionszone eingeführt werden mittels mindestens einer Gasbeschleunigungseinrichtung, die in die teilchenförmigen Elemente eingebettet ist, welche die Erzielung einer Gasgeschwindigkeit am Ausgang dieser Beschleunigungseinrichtung von mindestens 10 m/s erlaubt,

(b) man läßt das in der Stufe (a) erhaltene Gasgemisch in einer Reaktionszone aus Keramikmaterial reagieren, die umfaßt eine Vielzahl von Hohlräumen, die Durchgänge aufweisen, die in mindestens einer Richtung eine Dimension haben, die höchstens gleich dem Flammenhemmungsabstand ist, wobei der Abstand zwischen den Zonen höchstens gleich dem Flammenhemmungsabstand ist.

2. Verfahren nach Anspruch 1, in dem die Dimension der Durchgänge in der Reaktionszone größer ist als diejenige der Durchgänge in der Dispersionszone.

3. Verfahren nach Anspruch 1 oder 2, in dem das in der Stufe (a) erhaltene Gasgemisch in eine Mischzone aus Keramikmaterial eingeführt wird, die eine Vielzahl von Hohlräumen definiert, die Durchgänge aufweisen, die in mindestens einer Richtung eine Dimension haben, die höchstens gleich dem Flammenhemmungsabstand ist, wobei das in dieser Mischzone erhaltene Gasgemisch anschließend in die Reaktionszone eingeführt wird, wobei der Interzonen-Abstand zwischen der Dispersionszone und der Mischzone einerseits und zwischen der Mischzone und der Reaktionszone andererseits höchstens gleich dem Flammenhemmungsabstand der Flamme ist.

4. Verfahren nach Anspruch 3, in dem die Dimension der Durchgänge in der Reaktionszone einerseits gröber ist als die Dimension der Durchgänge in der Mischzone und in der Dispersionszone und andererseits gröber ist als der Abstand zwischen der Dispersionszone und der Mischzone.

5. Verfahren nach einem der Ansprüche 1 bis 4, in dem die Dispersionszone vollständig gefüllt ist mit teilchenförmigen Elementen aus einem Keramikmaterial, welche Dimensionen von $10^{-5}$ bis $3,5 \times 10^{-3}$ m haben.

6. Verfahren nach einem der Ansprüche 1 bis 5, in dem der Gasausgang der Beschleunigungseinrichtungen in einem Abstand von der an die Dispersionszone angrenzenden Zone angeordnet ist, der 0,1 bis 99,9 % des Wertes der größten Dimension der Dispersionszone entspricht.

7. Verfahren nach einem der Ansprüche 1 bis 6, in dem die Gasgeschwindigkeit am Ausgang der Beschleunigungseinrichtungen kontinuierlich so eingestellt wird, daß die Temperatur des Gasgemisches am Eingang in die Reaktionszone höchstens 800°C beträgt.

8. Verfahren nach einem der Ansprüche 1 bis 6, in dem die Oxidationsreaktion eingeleitet wird durch Erwärmung mindestens eines Teils der Reaktionszone, die an die Dispersionszone oder an die Mischzone angrenzt, auf eine Temperatur von 700 bis 1 200°C.

9. Oxidationsreaktor zur Durchführung des Verfahrens nach Anspruch 1, der umfaßt Einrichtungen zur Einführung eines oxidierenden Gases (5), Einrichtungen zur Einführung einer oxidierbaren Charge (6), Einrichtungen zum Abziehen der Reaktionsprodukte (13) und, mindestens auf einem Teil des Querschnitts des Reaktors, mindestens eine Reaktionseinrichtung (4) aus einem Keramikmaterial, die mit den Abzugseinrichtungen in Verbindung steht und eine Füllung aus einem Keramikmaterial enthält, die geeignet ist, in mindestens einem Teil dieser Einrichtung eine Vielzahl von Hohlräumen zu bilden, die Durchgänge (9) aufweisen, die in mindestens einer Richtung eine Dimension haben, die höchstens $10^{-2}$ m beträgt, wobei der Reaktor dadurch gekennzeichnet ist, daß er aufweist auf mindestens einem Teil seines Abschnitts Dispersionseinrichtungen (2) aus einem Keramikmaterial, die eine erste Füllung aus teilchenförmigen Elementen aus einem Keramikmaterial mit solchen Dimensionen enthält, daß die freien Durchgänge zwischen diesen Elementen mindestens in einer Richtung eine Dimension haben, die höchstens $10^{-2}$ m beträgt, wobei diese Elemente Dimensionen von $10^{-5}$ bis $3,5 \times 10^{-3}$ m haben, in die:

– entweder mindestens eine Beschleunigungseinrichtung (7a) eingebettet ist, die mit den Einrichtungen zur Zuführung des oxidierenden Gases (5) und der oxidierbaren Charge (6) verbunden ist,

– oder mindestens zwei Beschleunigungseinrichtungen (7a und 7b) eingebettet sind, von denen jede mit einer einzigen Zuführungseinrichtung verbunden ist, wobei mindestens eine der Beschleunigungseinrichtungen (7b) mit der Einrichtung zur Zuführung der oxidierbaren Charge (6) verbunden ist und mindestens eine der Beschleunigungseinrichtungen (7a) mit der Einrichtung zur Zuführung des oxidierenden Gases (5) verbunden ist, wobei der bestand zwischen den Dispersionseinrichtungen (2) und der Reaktionsein-

richtung (4) höchstens $10^{-2}$ m beträgt.

10. Reaktor nach Anspruch 9, dadurch gekennzeichnet, daß er umfaßt mindestens eine Mischeinrichtung (3) aus einem Keramikmaterial, die zwischen den Dispersionseinrichtungen und den Reaktionseinrichtungen angeordnet ist, die eine Vielzahl von Stufen (Böden) (8a, 8b, 8c) mit deutlich gegeneinander versetzten Maschen aufweist, wobei jede dieser Maschen auf der gesamten Länge der Mischeinrichtung (3) eine Vielzahl von Hohlräumen definiert, die Durchgänge aufweisen, die mindestens in einer Richtung eine Dimension haben, die höchstens $10^{-2}$ m beträgt, und wobei der Abstand zwischen den Dispersionseinrichtungen und der Mischeinrichtung einerseits und der Mischeinrichtung und den Reaktionseinrichtungen andererseits höchstens $10^{-2}$ m beträgt.

11. Reaktor nach Anspruch 9, dadurch gekennzeichnet, daß er umfaßt eine Mischeinrichtung (3) aus einem Keramikmaterial, die zwischen den Dispersionseinrichtungen und den Reaktionseinrichtungen angeordnet ist, die eine Vielzahl von im wesentlichen vertikalen Platten (16) aufweist, die diesseits und jenseits derselben eine Vielzahl von Vorsprüngen (17) und Kanälen (18) aufweisen, die diesseits und jenseits jeder Platte eine entgegengesetzte Neigung haben, wobei diese Platten so angeordnet sind, daß die Vorsprünge (17) und die Kanäle (18) sich kreuzen, wobei die Vielzahl von Hohlräumen, die durch diese Platten definiert sind, Durchgänge aufweisen, die in mindestens einer Richtung eine Dimension haben, die höchstens $10^{-2}$ m beträgt, und wobei der Abstand zwischen den Dispersionseinrichtungen und der Mischeinrichtung einerseits und zwischen der Mischeinrichtung und den Reaktionseinrichtungen andererseits höchstens $10^{-2}$ m beträgt.

12. Reaktor nach einem der Ansprüche 9 bis 11, dadurch gekennzeichnet, daß die Füllung der Reaktionseinrichtungen (4) mindestens einen Monolithen aus Keramik umfaßt, der mindestens zum Teil mit teilchenförmigen Elementen aus Keramik gefüllt ist.

13. Reaktor nach Anspruch 12, dadurch gekennzeichnet, daß der Monolith mindestens zum Teil mit teilchenförmigen Elementen aus Keramik gefüllt ist.

14. Reaktor nach einem der Ansprüche 9 bis 11, dadurch gekennzeichnet, daß die Füllung der Reaktionseinrichtungen (4) teilchenförmige Elemente aus Keramik mit Dimensionen von $10^{-5}$ bis $4 \times 10^{-3}$ m umfaßt, wobei die Dimensionen dieser teilchenförmigen Elemente in diesen Reaktionseinrichtungen größer sind als diejenigen der teilchenförmigen Elemente, welche die Dispersionseinrichtungen (2) füllen.

15. Reaktor nach einem der Ansprüche 9 bis 14, dadurch gekennzeichnet, daß die Füllung der Reaktionseinrichtungen in einem Teil mindestens dieser Einrichtungen mindestens einen Katalysator umfaßt.

16. Reaktor nach einem der Ansprüche 9 bis 15, dadurch gekennzeichnet, daß er umfaßt Einrichtungen (21) zur Messung der Temperatur, die in unmittelbarer Nähe zum Ende der Reaktionseinrichtungen auf der Seite, auf der das Gasgemisch ankommt, angeordnet sind, und Einrichtungen zur Regulierung der Durchflußmenge (23), die mit den Zuführungseinrichtungen (5 und 6) verbunden sind, wobei diese Meßeinrichtungen mit den Reguliereinrichtungen für die Zuführungsmengen des Gases so kooperieren, daß die Gasgeschwindigkeit am Ausgang der Beschleunigungseinrichtungen reguliert wird und die Temperatur am Eingang der Reaktionseinrichtungen auf einen Wert unterhalb eines vorgegebenen Wertes gehalten wird.

17. Reaktor nach einem der Ansprüche 9 bis 16, dadurch gekennzeichnet, daß er außerdem Erwärmungseinrichtungen in mindestens einem Teil der Reaktionseinrichtungen aufweist.

18. Verwendung des Reaktors nach einem der Ansprüche 9 bis 17 insbesondere für die Synthese von Methanol und höheren homologen Alkoholen aus Kohlenstoffoxiden und Wasserstoff.

## Claims

1. Method for oxidizing an oxidizable charge in the gas phase by means of a gas or a mixture of gases, including at least one oxidizing gas, comprising the following steps:

a) the oxidizable charge and the oxidizing gas are fed into a dispersion zone entirely filled with particulate elements made from ceramic material having dimensions such that the spaces or free passages between said elements have, in at least one direction, a dimension at most equal to the distance for quenching the flame which may result from oxidation of said charge by said oxidizing gas, said charge and said gas being fed into said dispersion zone through at least one gas acceleration means embedded in said particulate elements, making it possible to obtain a gas speed at the outlet of said acceleration means of at least 10m $\times$ s$^{-1}$, and

b) causing the gas mixture from step (a) to react in a ceramic material reaction zone comprising a multiplicity of spaces offering passages having, at least in one direction, a dimension at most equal to said flame quenching distance, the interzone distance being at most equal to said flame quenching distance.

2. The method as claimed in claim 1, wherein the dimension of the passages in said reaction zone is greater than that of the passages in said dispersion zone.

3. The method as claimed in claim 1 or 2, wherein the gas mixture from step (a) is fed into a ceramic material mixing zone defining a multiplicity of spaces providing passages having, in at least one direction, a dimension at most equal to said flame quenching distance, the gas mixture from this mixing zone then being fed into the reaction zone, the interzone distance between the dispersion zone and the mixing zone on the one hand and between the mixing zone and the reaction zone on the other being at most equal to said flame quenching distance.

4. The method as claimed in claim 3, wherein the dimension of the passages in said reaction zone is greater both than the dimension of the passages in said mixing zone and in said dispersion zone and greater than the distance between said dispersion zone and said mixing zone.

5. The method as claimed in one of claims 1 to 4, wherein said dispersion zone is entirely filled with ceramic material particulate elements having dimensions of $10^{-5}$ to $3.5 \times 10^{-3}$m.

6. The method as claimed in one of claims 1 to 5, wherein the gas outlet of said acceleration means is situated at a distance from the zone adjacent the dispersion zone by 0.1% to 99.9% of the value of the largest dimension of said dispersion zone.

7. The method as claimed in one of claims 1 to 6, wherein the gas speed at the outlet of said acceleration means is continuously regulated so that the temperature of the gas mixture at the inlet to the reaction zone is at most equal to 800°C.

8. The method as claimed in one of claims 1 to 6, wherein said oxidation reaction is initiated by heating to a temperature of 700 to 1200°C at least a part of the reaction zone adjacent the dispersion zone or the mixing zone.

9. An oxidation reactor for implementing the method as claimed in claim 1, comprising oxidizing fas feed means (5) and oxidizable charge feed means (6) and means (13) for discharging the reaction products and, over at least a part of the cross section of said reactor, at least one reaction means, (4) made from a ceramic material, connected to said discharge means and comprising a ceramic material packing adapted to define in at least a part of said means a multiplicity of spaces providing passages (9) with, in at least one direction, a dimension at most equal to $10^{-2}$m, being chacterized in that it said reactor comprising, at least over a part of its cross-section, ceramic material dispersion means (2) including a first packing of ceramic material particulate elements having dimensions such that the free passages between said elements have, in at least one direction, a dimension at most equal to $10^{-2}$m, said elements having dimensions of $10^{-5}$ to $3.5 \times 10^{-3}$m in which :

– either at least one acceleration means (7a) is embedded connected to the oxidizing gas and oxidizable charge feed means (6),

– or at least two acceleration means (7a, 7b) are embedded, each being connected to a single feed means, at least one of said acceleration means (7b) being connected to the oxidizable charge feed means (6) and at least one of said acceleration means (7a) being connected to the oxidizing gas feed means (5), the distance between said dispersion means (2) and said reaction means (4) being at most equal to $10^{-2}$ meter.

10. The reactor as claimed in claim 9, comprising at least one ceramic material mixing means (3) situated between said dispersion means and said reaction means, having a plurality of stages (8a, 8b, 8c) with substantially offset meshes, each of which defines over the whole length of said mixing means (3) a multiplicity of spaces providing passages with, at least in one direction, a dimension at most equal to $10^{-2}$m and the distance between said dispersion means and said mixing means on the one hand and between said mixing means and said reaction means on the other being at most equal to $10^{-2}$m.

11. The reactor as claimed in claim 9, comprising a ceramic material mixing means (3) situated between said dispersion means and said reaction means, having a plurality of substantially vertical plates (16) with, on the each side of each of them, a plurality of projections (17) and ducts (18) slanted oppositely on each side of each plate, said plates being disposed so that the projections (17) and the ducts (18) are crossed, the multiplicity of spaces defined by said plates having passages with, in at least one direction, a dimension at most equal to $10^{-2}$m, the distance between said dispersion means and said mixing means on the one hand and between said mixing means and said reaction means on the other being at most equal to $10^{-2}$m.

12. The reactor as claimed in one of claims 9 to 11, wherein the packing of said reaction means (4) comprises at least one ceramic monolith with a plurality of juxtaposed channels.

13. The reactor as claimed in claim 12, wherein said monolith is filled at least partially with ceramic particulate elements.

14. The reactor as claimed in one of claims 9 to 11, wherein the packing of said reaction means (4) comprises ceramic particulate elements having dimensions of $10^{-5}$ to $4 \times 10^{-3}$m, the dimensions of said particulate elements in said reaction means being greater than those of the particulate elements filling said dispersion means (2).

15. The reactor as claimed in one of claims 9 to 14, wherein the packing of said reaction means includes in a part at least of said means at least one catalyst.

16. The reactor as claimed in one of claims 9 to 15, comprising temperature measurement means (21) situated in the immediate vicinity of the end of said reaction means on the gas mixture intake side and flow regulation means (23) connected to said feed means (5, 6), said measurement means cooperating with said gas feed regulation means so as to regulate the speed of the gases at the outlet of said acceleration means and to maintain the temperature at the inlet of said reaction means below a predetermined value.

17. The reactor as claimed in one of claims 9 to 16, comprising means for heating at least a part of said reaction means.

18. Use of the reactor as claimed in one of claims 9 to 17, particularly for the synthesis of methanol and homologous upper alcohols from carbon oxides and hydrogen.

FIG.1A

FIG.1B

FIG.2A

FIG.2B

**FIG.3A**

8a

15

**FIG.3E**

10

13

2

8a

8b

8c

21

14

**FIG.3B**

8b

15

**FIG.4**

45°

16

17

18

**FIG.3C**

b

8a

a

8b

8a

8b

**FIG.3D**

**FIG.5**

9

4

18